# FASCICULE DE BREVET EUROPEEN

(11) **EP 1 237 745 B1**
(45) Date de publication et mention de la délivrance du brevet: **09.09.2009**
(21) Numéro de dépôt: 01978518.7
(22) Date de dépôt: 09.10.2001
(51) Int. Cl.: B60H 3/00, A61L 9/12

(54) **DISPOSITIF DE CHAUFFAGE ET DE CLIMATISATION COMPORTANT DES MOYENS D'ODORISATION DE L'HABITACLE D'UN VEHICULE**
HEIZUNG ODER KLIMAANLAGE MIT DUFTDIFFUSIONSMITTEL FÜR EINEN FAHRZEUGINNENRAUM
HEATING AND AIR CONDITIONING DEVICE COMPRISING FRAGRANCE-SUPPLYING MEANS IN A VEHICLE PASSENGER COMPARTMENT

(30) Priorité: 17.10.2000 FR 0013282
(43) Date de publication de la demande: 11.09.2002
(73) Titulaire: Valeo Systèmes Thermiques, 78321 Le Mesnil St Denis Cedex (FR)
(72) Inventeur: FEUILLARD, Vincent, F-78320 Le Mesnil Saint Denis (FR); LEMAITRE, Nathalie, F-78220 Viroflay (FR)
(86) Numéro de dépôt international: PCT/FR2001/003114
(87) Numéro de publication internationale: WO 2002/032707

(56) Documents cités:
- EP-A- 0 325 468
- US-A- 4 813 344
- US-A- 5 297 988

## Description

L'invention concerne les dispositifs d'odorisation de l'habitacle d'un véhicule automobile.

L'intérieur d'une voiture constitue un espace clos. En été et en hiver, lorsque les fenêtres de la voiture sont fermées et que le système de conditionnement d'air est utilisé pour refroidir ou chauffer l'intérieur de l'habitacle, la transpiration corporelle du conducteur et des passagers, ainsi que la fumée de cigarette qui s'accumule dans l'habitacle et dans les conduits du boîtier de chauffage et du système de climatisation d'air donnent naissance à des odeurs désagréables. Ces odeurs peuvent aussi provenir de l'extérieur du système de climatisation, par exemple de gaz d'échappement, ou encore des matières plastiques et colles de l'habitacle. En conséquence, il est connu d'équiper les véhicules d'appareils qui ont pour fonctions principales d'éliminer ou de masquer les odeurs, mais aussi de procurer d'autres effets, par exemple physiologiques, etc. L'invention concerne un dispositif de ce type.

Plus précisément, l'invention concerne un dispositif de chauffage et/ou de climatisation de l'habitacle d'un véhicule comprenant un boîtier ayant une extrémité amont, et une extrémité aval débouchant dans l'habitacle, un ventilateur pour aspirer un flux d'air dans le boîtier par l'extrémité amont et pour le souffler dans l'habitacle par l'extrémité aval du boîtier, des moyens d'odorisation comprenant une enveloppe contenant une quantité d'un ou de plusieurs agents aromatiques fixés sur un support logé dans l'enveloppe, et aptes à dégager une fragrance, l'enveloppe comportant une zone de diffusion de l'agent aromatique percée d'au moins un orifice de passage pour permettre le passage du flux d'air à travers l'enveloppe. Le terme vapeur aromatique entend désigner ici une substance parfumée apte à dégager une vapeur aromatique communément appelée fragrance.

On connaît déjà des dispositifs d'odorisation de ce type. Le document japonais KOKAI N° 58-81817 décrit un dispositif selon le préambule de la revendication 1 avec un dispositif d'odorisation comprenant une cassette contenant un agent aromatique volatil situé en dérivation du boîtier du dispositif de chauffage et de climatisation.

Les brevets US 5 429 180 et 5 297 988 décrivent chacun un dispositif d'odorisation de l'habitacle d'un véhicule comportant des circuits logiques de commande du débit d'agent aromatique dans le boîtier.

Ces dispositifs connus présentent l'inconvénient d'être complexes tant à fabriquer qu'à utiliser.

La présente invention remédie à ces inconvénients. Elle propose un dispositif de chauffage et de climatisation de l'habitacle comportant des moyens d'odorisation simples à fabriquer et à utiliser.

A cet effet, le support de l'agent aromatique ou des agents aromatiques est mobile entre au moins une position de diffusion dans laquelle l'agent aromatique ou l'un des agents aromatiques est en regard de la zone de diffusion, et une position de stockage dans laquelle l'agent aromatique est isolé de la zone de diffusion par une barrière étanche à la fragrance, le dispositif comportant une commande qui permet à un opérateur situé dans l'habitacle du véhicule de commander le déplacement du support mobile entre la position de diffusion et la position de stockage.

Ainsi, le dispositif d'odorisation de l'invention est simple à fabriquer. Il ne comporte que des pièces mécaniques à l'exception de tout circuit de commande logique. D'autre part, il est simple à utiliser, la mise en marche et l'arrêt du dispositif pouvant être obtenus manuellement, à partir du tableau de bord, au moyen d'un bouton rotatif, d'un curseur ou d'un bouton poussoir. De la même manière, si le dispositif comporte plusieurs agents aromatiques, le choix de l'agent aromatique peut être effectué par l'intermédiaire du même bouton rotatif, curseur ou bouton poussoir.

Selon une variante de réalisation, le support est mobile en translation. Dans une réalisation particulière, l'enveloppe est constituée par un tube et le support mobile par une tige montée coulissante dans le tube, au moins un agent aromatique étant monté sur la tige coulissante, et au moins un disque de séparation formant une barrière étanche à la vapeur de l'agent aromatique étant prévue sur la tige coulissante pour isoler l'agent aromatique ou les agents aromatiques de la zone de diffusion.

Dans une autre variante de réalisation, le support est mobile en rotation. Dans une réalisation particulière, le support tournant est un disque comportant une pluralité de logements espacés angulairement, chaque logement étant apte à contenir un agent aromatique.

Dans une autre variante de réalisation, le support tournant est un cylindre comportant des perforations selon deux zones opposées et monté tournant autour d'un axe longitudinal, ce cylindre contenant une quantité d'au moins un agent aromatique, et l'enveloppe comportant au moins deux orifices diamétralement opposés constituant une zone de diffusion de l'agent aromatique, le support tournant pouvant être déplacé angulairement entre une position de diffusion, dans laquelle les perforations du cylindre sont en regard de la zone de diffusion de l'enveloppe et une position de stockage dans laquelle les perforations du cylindre sont décalées angulairement de la zone de diffusion.

Fréquemment, le boîtier de chauffage et/ou de climatisation comporte un filtre à air qui permet de filtrer l'air atmosphérique aspiré à l'extérieur du véhicule. Dans un tel cas, l'enveloppe est avantageusement intégrée dans le filtre à air.

De préférence, chaque agent aromatique se présente sous la forme d'une cartouche solide. De telles cartouches sont en effet plus faciles à manipuler.

Dans une réalisation particulière, le dispositif comporte un module situé au tableau de bord du véhicule et intégrant l'enveloppe, cette dernière étant reliée au boîtier par une canalisation d'entrée d'air raccordée en aval du ventilateur, et par une canalisation de sortie raccordée en aval de la canalisation d'entrée d'air. De préférence, la canalisation de sortie d'air est raccordée à une zone de mixage du boîtier du dispositif de chauffage et/ou de climatisation.

Avantageusement encore, le module comporte un trou d'accès en façade accessible au conducteur ou à un passager du véhicule, ce trou d'accès permettant le remplacement de la cartouche ou des cartouches d'agent aromatique.

Selon une autre caractéristique de l'invention, le dispositif comprend des moyens d'affichage de l'agent aromatique sélectionné.

D'autres caractéristiques et avantages de la présente invention apparaîtront encore à la lecture de la description qui suit d'exemples de réalisation donnés à titre illustratif en référence aux figures annexées. Sur ces figures :
- la figure 1 est une vue schématique générale d'un dispositif de chauffage et/ou de climatisation conforme à l'invention ;
- la figure 2 est une vue de détail des moyens d'odorisation du dispositif représenté sur la figure 1 ;
- la figure 3 est une vue schématique générale, similaire à la figure 1, d'une variante de réalisation de l'invention ;
- les figures 4a et 4b sont respectivement une vue de face et une vue en élevation d'un support mobile en forme de disque pouvant être utilisé dans la variante de réalisation du dispositif représenté sur la figure 3 ;
- la figure 5 est une vue d'un filtre à air intégrant un cylindre mobile contenant un agent aromatique ;
- la figure 6 est une vue en section, à échelle agrandie, du cylindre représenté sur la figure 5 ; et
- les figures 7 à 10 représentent différents moyens d'affichage et/ou de reconnaissance de l'agent aromatique sélectionné.

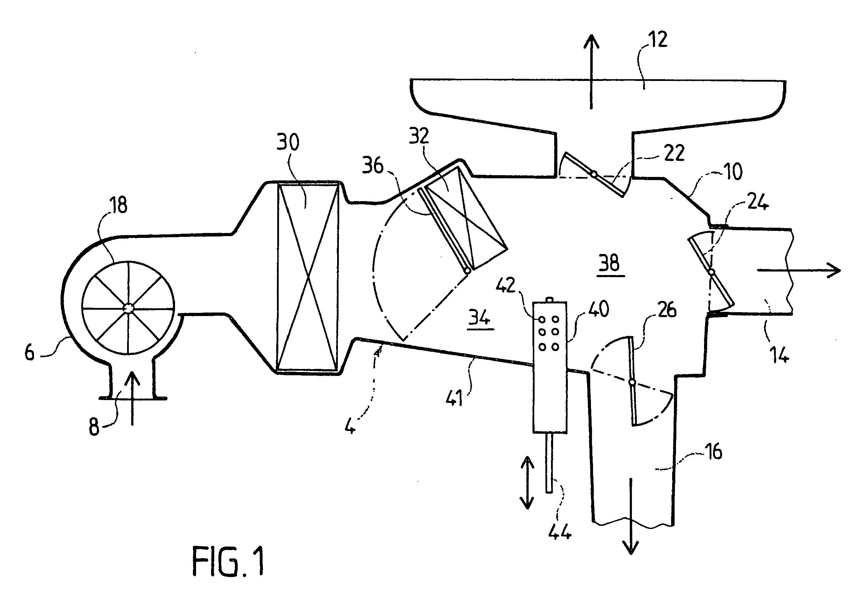

Sur la figure 1, le boîtier 4 du dispositif de chauffage comporte une extrémité amont 6 comportant une ouverture d'entrée d'air 8 et une extrémité aval 10 comportant trois sorties d'air débouchant dans l'habitacle du véhicule, à savoir respectivement une sortie d'air 12 de dégivrage de pare-brise, une sortie d'air 14 de ventilation et/ou de chauffage de l'habitacle et une sortie 16 pour le chauffage des pieds du conducteur et des passagers. Un ventilateur 18 entraîné par un moteur électrique (non représenté) aspire l'air par l'entrée 8 et crée un flux d'air dans le boîtier 4. Des volets 22, 24 et 26 permettent de régler la quantité d'air qui passe par chacune des sorties d'air 12, 14 et 16 respectivement.

Un évaporateur 30 faisant partie d'un circuit de climatisation, est monté dans le boîtier de chauffage 4 immédiatement en aval du ventilateur 18. Le flux d'air est refroidi lorsqu'il passe au travers de l'évaporateur 30.

Un radiateur de chauffage 32 intégré dans le circuit de refroidissement du moteur du véhicule (non représenté) est également monté dans le boîtier de chauffage 4. Le flux d'air qui traverse le boîtier est réchauffé lorsqu'il traverse le radiateur de chauffage 32. Le radiateur 32 est monté dans le boîtier 4 de telle manière qu'un passage de contournement 34 qui évite le radiateur de chauffage 32 est prévu.

Un volet de mixage d'air 36 est prévu du côté amont du radiateur de chauffage 32. Ce volet peut être déplacé entre une position représentée en trait plein ouvrant le passage de contournement 34 et empêchant le flux d'air de traverser le radiateur de chauffage 32, et une position représentée en trait mixte interdisant le passage du flux d'air par le passage de contournement 34 et le contraignant à traverser ce radiateur, de sorte que l'air s'échauffe.

Dans une position intermédiaire, le rapport entre le flux d'air qui traverse le radiateur 32 et le flux d'air qui traverse le passage de contournement 34 est réglé par le degré d'ouverture du volet 36 afin de régler la température de l'air lorsque le flux d'air chaud et le flux d'air froid se mélangent dans une zone de mixage 38. En variante, il est possible d'utiliser un appareil dit "à mixage sur l'eau", dans lequel le débit de liquide de refroidissement qui travers le radiateur de chauffage est réglable, ce qui permet de régler la température du flux d'air issu du radiateur.

Une enveloppe 40 présentant la forme générale d'un cylindre allongé traverse une paroi 41 du boîtier de chauffage 4. L'enveloppe 40 comporte une partie située à l'intérieur de la zone de mixage 38 et une partie située à l'extérieur du boîtier 4. La partie de l'enveloppe 40 située dans la zone de mixage est percée d'orifices 42 formant une zone de diffusion. A l'extérieur du boîtier, on trouve une tige 44 constituant un moyen de commande. Dans une variante, l'enveloppe 40 pourrait être logée complètement à l'intérieur de la paroi 41 et montée sur une tige extérieure 44.

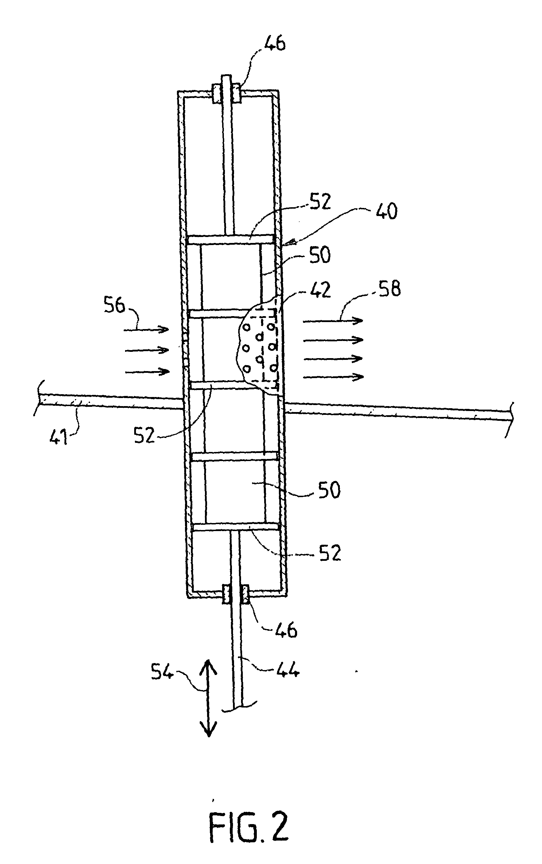

On a représenté sur la figure 2 une vue à échelle agrandie de l'enveloppe 40 montée sur la paroi 41. L'enveloppe 40 est traversée de part en part par une tige 44 montée coulissante sur deux paliers 46 situés à chacune des extrémités de l'enveloppe. Plusieurs cartouches d'agent aromatique ou parfum, trois dans l'exemple de réalisation représenté, sont montées sur la tige 44. Un emplacement sans cartouche est prévu pour constituer une position de stockage ou d'arrêt. Il est toutefois possible de supprimer cette position d'arrêt en ajoutant une quatrième cartouche de parfum dans l'emplacement vide précité. Chaque cartouche 50 est placé entre deux disques 52, également montés sur la tige 44 et formant une liaison glissante étanche avec la paroi cylindrique intérieure de l'enveloppe 40.

Les cartouches d'agent aromatique 50 sont de préférence solides ; on peut avoir autant de parfums que de cartouches, ou bien on peut prévoir deux cartouches de chaque parfum, afin de permettre le remplacement d'une cartouche lorsque la précédente est épuisée. Il est envisageable aussi de prévoir plusieurs cartouches du même parfum. La tige 44 peut être déplacée alternativement en translation comme symbolisé par la flèche 54 par des moyens de commande (non représentés). Le déplacement de la tige 44 a pour effet de placer l'une des cartouches d'agent aromatique en regard des perforations 42 pratiquées dans une zone de diffusion de l'enveloppe. En dehors de cette zone de diffusion, l'enveloppe 40 ne comporte aucune ouverture.

Lorsqu'une cartouche 50 d'agent aromatique est placée dans la zone de diffusion, comme représenté sur la figure 2, les deux disques 52 situés de part et d'autre de la cartouche d'agent aromatique forment une chambre étanche à l'air et à la fragrance de l'agent aromatique. Etant donné que l'enveloppe est placée sur le trajet du flux d'air mis en mouvement par le ventilateur 18 du boîtier de chauffage 4, l'air pénètre par les perforations 42, comme schématisé par les flèches 56, traverse la chambre définie entre les deux disques 52 en entraînant avec lui la vapeur de l'agent aromatique. L'air sort alors par le côté opposé de l'enveloppe comme représenté par les flèches 58, puis il pénètre dans l'habitacle par l'une des sorties d'air 12, 14 et 16 (voir figure 1). On odorise ainsi l'habitacle du véhicule. Le changement de parfum se fait aisément par un simple déplacement de la tige 44 qui permet d'amener un autre agent aromatique en regard des perforations 42 de la zone de diffusion de l'enveloppe. Il est également possible de ne diffuser aucun parfum en ne mettant aucune cartouche d'agent aromatique dans la zone de diffusion.

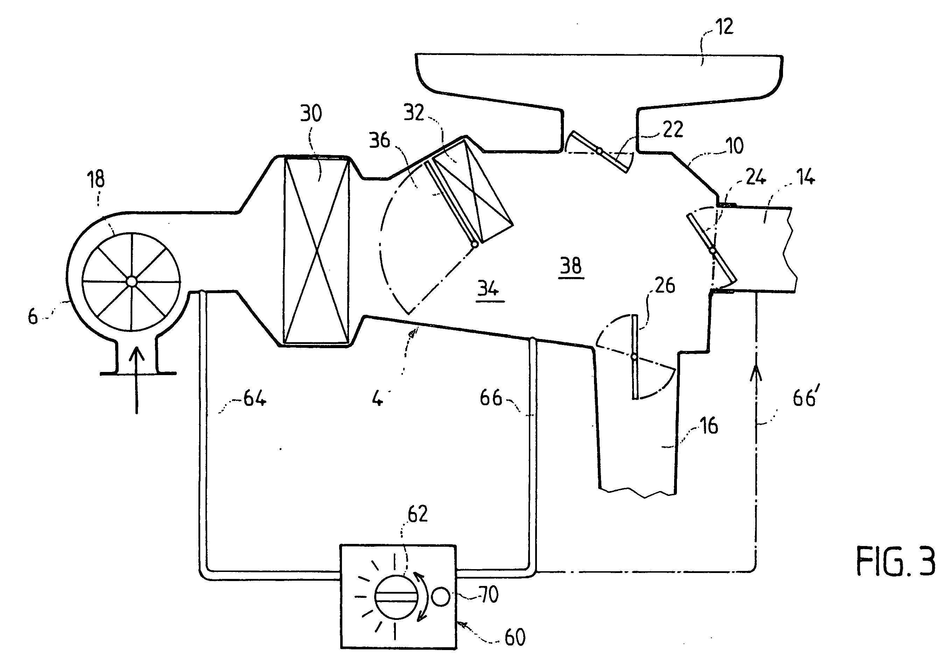

On a représenté sur la figure 3 une variante de réalisation d'un dispositif conforme à l'invention semblable à celui qui a été décrit en référence aux figures 1 et 2. Les éléments identiques ont été désignés par des numéros de référence identiques. Cette variante se distingue par le fait qu'elle comporte un module 60 monté au tableau de bord du véhicule et accessible au conducteur et aux passagers. L'enveloppe (non représentée) qui contient les cartouches de parfum est disposée à l'intérieur du module 60, juste derrière la façade du tableau de bord. Une canalisation d'entrée d'air 64 relie une partie du boîtier de chauffage 4 à l'enveloppe, tandis qu'une canalisation de sortie d'air 66 relie cette enveloppe à une partie du boîtier située en aval de la première. Le module 60 peut être placé en un autre endroit accessible pour la rechange, par exemple sous la planche de bord.

Dans l'exemple de réalisation décrit, la canalisation d'entrée d'air 64 est raccordée au boîtier 4 juste après le ventilateur 18, en amont de l'évaporateur 30. La canalisation de sortie d'air 66 débouche au niveau de la zone de mixage 38. Les canalisations 64 et 66 forment ainsi une dérivation au circuit de chauffage et de climatisation principal. Une partie du flux d'air mis en mouvement par le ventilateur 18 traverse cette dérivation en entraînant avec lui la fragrance de l'agent aromatique qui est disposé dans la zone de diffusion de l'enveloppe. Cet air est amené jusqu'à la zone de mixage 38 avant de pénétrer dans l'habitacle par l'une des trois sorties d'air 12, 14 et 16, comme décrit précédemment. Dans une variante de réalisation, la canalisation de sortie d'air 66 peut être raccordée directement à la sortie d'air de ventilation 14.

Cette réalisation est avantageuse parce que l'air chargé de la fragrance d'agent aromatique ne traverse pas l'évaporateur. En effet, lorsque le flux d'air traverse l'évaporateur, une partie de la vapeur qu'il contient se condense par suite de son refroidissement. Le liquide qui résulte de cette condensation est évacué à l'extérieur du véhicule. Si l'air chargé d'agent aromatique traversait l'évaporateur, une partie de ce dernier serait évacué vers l'extérieur en pure perte. La fragrance piégée en surface de l'évaporateur peut créer une rémanence qui reste perceptible même lorsque l'odorisation est en position d'arrêt.

Dans l'exemple de réalisation représenté sur la figure 3, le support des cartouches d'agent aromatique est monté mobile en rotation. Un bouton 62, accessible au tableau de bord, est monté directement sur l'axe de rotation du support mobile des parfums. Par une simple rotation, le conducteur du véhicule ou un passager peut sélectionner l'une des cartouches de parfum disponibles, une rechange de cartouche lorsque la cartouche précédente est épuisée, ou encore arrêter le dispositif d'odorisation en ne sélectionnant aucune cartouche. Le module 60 comporte encore un trou d'accès 70 qui permet un remplacement aisé des cartouches de parfum depuis l'habitacle du véhicule. Il suffit de tourner le bouton rotatif 62 pour amener la cartouche à remplacer en face du trou d'accès 70, de la retirer et de placer une cartouche neuve.

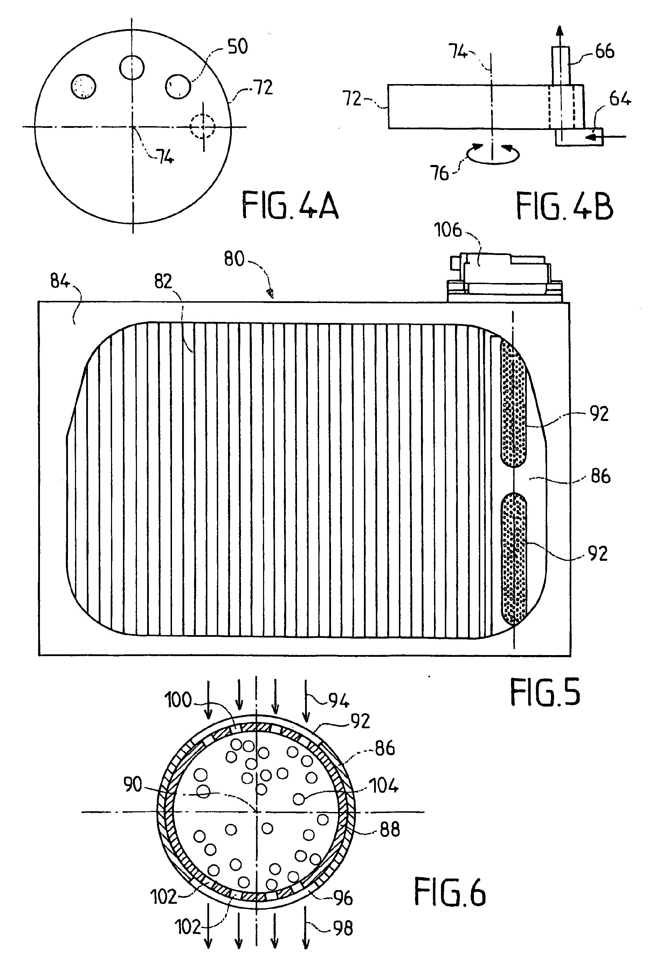

On a représenté sur les figures 4a et 4b une vue de face et une vue en élévation du support des cartouches d'agent aromatique 50. Ce support 72 présente la forme d'un disque épais dans lequel sont prévus des logements circulaires espacés angulairement pour recevoir plusieurs cartouches de parfum. L'un des emplacements ne comporte aucune cartouche pour permettre l'arrêt du dispositif d'odorisation. Le support 72 est mobile en rotation, comme schématisé par la flèche 76, autour d'un axe 74 représenté en traits mixtes. Le flux d'air pénètre par la canalisation d'amenée d'air 64, traverse le disque en entraînant les fragrances de l'agent aromatique et ressort par la canalisation de sortie d'air 66, comme décrit précédemment en référence à la figure 3.

On a représenté sur les figures 5 et 6 une troisième variante de réalisation de l'invention. En général, l'air aspiré dans le boîtier de chauffage est filtré parce qu'il provient de l'atmosphère extérieure et qu'il est par conséquent chargé de poussières et de particules. Cet air traverse donc un filtre anti-poussières constitué d'un média non-tissé 82 en fibres synthétiques ou cellulosiques, et disposé dans un support en matière plastique ou métallique 84.

Conformément à l'invention, on a intégré un dispositif d'odorisation dans ce filtre. Il comporte deux cylindres coaxiaux, à savoir un premier cylindre fixe 86 et un second cylindre 88 monté tournant à l'intérieur du cylindre 86 autour d'un axe 90 (voir figure 6). Le cylindre 86 est percé d'une ou de plusieurs lumières 92 situées du côté de l'entrée du flux d'air, schématisée par les flèches 94. Il comporte d'autre part une ou plusieurs lumières 96 diamétralement opposées à la ou aux lumières 92 pour permettre la sortie du flux d'air, comme schématisé par les flèches 98. Le cylindre mobile 88 comporte une première zone pourvue de perforations 100 et une seconde zone diamétralement opposée à la première pourvue de perforations 102. A l'intérieur du cylindre 88 est logé un agent aromatique qui se présente par exemple sous la forme de billes ou microcapsules 104. Lorsque le cylindre 88 se trouve dans la position angulaire représentée sur la figure 6, l'air pénètre par les perforations 100, traverse le cylindre 88 en circulant à travers les billes ou microcapsules 104 et ressort par les perforations diamétralement opposées 102 en traversant la lumière 96 du cylindre fixe 86. De la même manière que précédemment, le flux d'air entraîne les fragrances d'agent aromatique dégagées par les billes ou microcapsules 104.

Le cylindre 88 peut être tourné angulairement de 90° de telle manière que les perforations 100 et 102 se trouvent en face d'une zone du cylindre fixe 86 qui ne comporte pas d'ouvertures et que les deux zones du cylindre mobile 88 situées entre les zones perforées 100 et 102 se trouvent en regard des lumières 92 et 96. La rotation du cylindre peut également être de 60° ou 45° pour permettre de faire varier l'intensité dans le cas d'une seule fragrance, ou pour offrir le choix entre 2 ou 3 fragrances. Ainsi, l'agent aromatique contenu dans le cylindre 88 est isolé de manière étanche du flux d'air et le dispositif d'odorisation est arrêté. Un motoréducteur 106 monté sur le support 84 du filtre permet l'entraînement en rotation du cylindre mobile 88 entre ces deux positions.

On constate que dans cette variante de réalisation, une seule fragrance est disponible et que le seul réglage possible est la mise en marche ou l'arrêt du dispositif. Le motoréducteur 106 est commandé électriquement depuis la planche de bord du véhicule.

Selon l'invention, il est avantageux que le support 44, 72 ou 88 de l'agent aromatique soit situé après l'évaporateur 30, après le radiateur de chauffage 32, et avant les sorties d'air 12, 14 et 16 débouchant dans l'habitacle du véhicule.

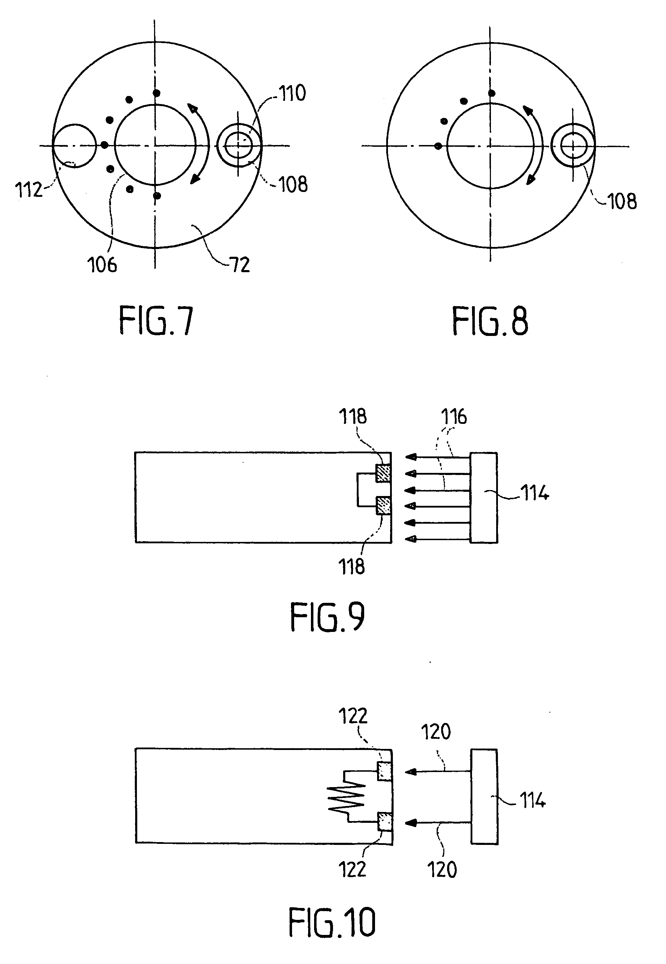

Dans la forme de réalisation de la figure 7, le support 72 s'apparente à celui des figures 4a et 4b. Il se présente sous la forme d'un disque dans lequel sont prévus des logements circulaires pour recevoir des cartouches de parfums.

Ce support est propre à être entraîné en rotation par un bouton 106 accessible depuis la planche de bord (non représenté) du véhicule. Cette planche de bord comporte une fenêtre transparente 108 qui permet de visualiser un symbole 110 (par exemple une pastille de couleur) identifiant l'agent aromatique utilisé. Par ailleurs, la planche de bord comporte une trappe d'accès 112 qui permet de remplacer une cartouche utilisée.

On peut ainsi afficher directement un symbole représentatif de l'agent aromatique utilisé.

La figure 8 constitue une variante de la forme de réalisation précédente à la différence près que la fenêtre 108 sert aussi de trappe d'accès pour le remplacement d'une cartouche. Il en résulte que la fenêtre 112 de la figure 7 est purement et simplement supprimée.

Dans certains cas, la cartouche n'est pas directement placée derrière la planche de bord, et on prévoit des moyens d'affichage et de reconnaissance à distance.

On peut prévoir par exemple un logement correspondant à un agent aromatique déterminé, la reconnaissance de la cartouche s'effectuant par exemple par des moyens de détrompage.

Dans la forme de réalisation de la figure 9, la reconnaissance d'une cartouche parfumée s'effectue par des moyens électroniques comprenant un détecteur 114 avec des lignes de contact 116 pour reconnaître des contacts 118 de la cartouche par continuité électrique.

Dans le cas de la figure 10, le détecteur 114 comporte deux contacts 120 susceptibles de coopérer avec deux contacts 122 d'une cartouche parfumée.

Bien entendu, il est possible d'envisager d'autres types de moyens pour reconnaître et afficher le type de l'agent aromatique sélectionné.

L'agent aromatique de l'invention peut être un parfum liquide imprégné sur un support approprié qui est avantageusement choisi parmi les craies, les argiles, les papiers, les buvards, les média tissés ou non-tissés, les sachets de pot-pourri, les billes en matière plastique (par exemple formées d'un mélange de polyéthylène et de polypropylène), les billes de bois ou de verre, etc.

En variante, l'agent aromatique peut être mélangé en tant que constituant avec des supports tels que des gels, des microcapsules, des poudres, des éléments en matière plastique, etc.

La nature chimique de l'agent aromatique dépend de l'objectif recherché dans l'odorisation du véhicule. On peut en particulier procurer l'un au moins des effets suivants.

Effet physiologique : apaisant, relaxant, tonifiant, etc. Exemple : odeur zestée (limonène, citral) ou terpénique (camphre, menthol).

Effet d'image : signature olfactive d'une marque de véhicule ou d'un style de véhicule (luxe, sport, familiale, etc.). Exemples : odeur boisée masculine (acétate de vétyvéryle, ambroxan) ou odeur ludique douce (benzoate de benzyle, vanilline, coumarine, trans-anéthol) ou fruitée (undécalone, citral).

Effet de mode : en rapport avec les fragrances utilisées en parfumerie fine ou technique du moment.

Effet personnalisé : offrir un large éventail à l'utilisateur selon son goût.

On a ainsi réalisé un dispositif de chauffage et de climatisation comportant des moyens d'odorisation simples à fabriquer, à intégrer et à utiliser, et comportant, dans certaines variantes, plusieurs parfums ou fragrances qui peuvent être sélectionnés aisément.

## Revendications

1. Dispositif de chauffage et/ou de climatisation de l'habitacle d'un véhicule automobile, comprenant un boîtier (4) ayant une extrémité amont (6) et une extrémité aval (10) débouchant dans l'habitacle, un ventilateur (18) pour aspirer un flux d'air dans le boîtier (4) par l'extrémité amont et pour le souffler dans l'habitacle par l'extrémité aval du boîtier (4), des moyens d'odorisation comprenant une enveloppe (40) contenant une quantité d'un ou de plusieurs agents aromatiques (50) fixés sur un support (44, 72, 88) logé dans l'enveloppe (50, 86) et apte à dégager une fragrance, l'enveloppe (50, 86) comportant une zone de diffusion de l'agent aromatique percée d'au moins un orifice de passage (42, 92, 96) pour permettre le passage du flux d'air (56, 58) à travers l'enveloppe,
**caractérisé en ce que** le support (44, 72, 88) de l'agent aromatique est mobile entre au moins une position de diffusion dans laquelle l'agent aromatique ou l'un des agents aromatiques est en regard de la zone de diffusion, et une position de stockage dans laquelle l'agent aromatique est isolé de la zone de diffusion par une barrière étanche (52) à la fragrance de l'agent aromatique, le dispositif comportant une commande (44) qui permet à un opérateur situé dans l'habitacle du véhicule de commander le déplacement du support mobile (44, 72, 88) entre la position de diffusion et la position de stockage.

2. Dispositif selon la revendication 1, **caractérisé en ce que** le support (44) est mobile en translation.

3. Dispositif selon la revendication 2, **caractérisé en ce que** l'enveloppe est constituée par un tube (40) et le support mobile par une tige (44) montée coulissante dans le tube (40), au moins un agent aromatique (50) étant monté sur la tige coulissante (44), et au moins un disque de séparation (52) formant une barrière étanche à la vapeur de l'agent aromatique étant prévu sur la tige coulissante (44) pour isoler l'agent aromatique ou les agents aromatiques de la zone de diffusion.

4. Dispositif selon la revendication 1, **caractérisé en ce que** le support (72) est mobile en rotation.

5. Dispositif selon la revendication 4, **caractérisé en ce que** le support tournant est un disque (72) comprenant une pluralité de logements espacés angulairement, chaque logement étant apte à contenir un agent aromatique (50).

6. Dispositif selon la revendication 5, **caractérisé en ce que** le support tournant est un cylindre (88) comprenant des perforations (100, 102) selon deux zones opposées et monté tournant autour d'un axe longitudinal (90), ce cylindre contenant une quantité d'au moins un agent aromatique, et **en ce que** l'enveloppe (86) comporte au moins deux orifices (92, 96) diamétralement opposés constituant une zone de diffusion de l'agent aromatique, le support tournant (88) pouvant être déplacé angulairement entre une position de diffusion dans laquelle les perforations du cylindre sont en regard de la zone de diffusion de l'enveloppe, et une position de stockage dans laquelle les perforations du cylindre (88) sont décalées angulairement de la zone de diffusion.

7. Dispositif selon la revendication 6, **caractérisé en ce qu'**il comporte un filtre à air (80), et **en ce que** l'enveloppe (86) est intégrée dans le filtre à air (80).

8. Dispositif selon l'une des revendications 1 à 7, **caractérisé en ce que** l'agent aromatique se présente sous la forme d'une cartouche solide.

9. Dispositif selon l'une des revendications 1 à 8, **caractérisé en ce qu'**il comporte un module (60) situé au tableau de bord du véhicule et intégrant l'enveloppe (40), cette dernière étant reliée au boîtier (4) par une canalisa tion d'entrée d'air (64) raccordée en aval du ventilateur (18) et par une canalisation de sortie d'air (66) raccordée au boîtier (4) en aval de la canalisation d'entrée d'air (64).

10. Dispositif selon la revendication 9, **caractérisé en ce que** la canalisation de sortie d'air (66) est raccordée à une zone de mixage (38) du boîtier (4) du dispositif de chauffage.

11. Dispositif selon l'une des revendications 7 à 10, **caractérisé en ce que** le module (60) comporte un trou d'accès en façade (70) accessible au conducteur ou à un passager du véhicule, ce trou d'accès (70) permettant le remplacement des cartouches (50) d'agent aromatique.

12. Dispositif selon l'une des revendications 1 à 11, **caractérisé en ce qu'**il comprend des moyens d'affichage (108, 110 ; 114) de l'agent aromatique sélectionné.

13. Dispositif selon l'une des revendications 1 à 12, **caractérisé en ce qu'**il comprend en outre un évaporateur (30) et **en ce que** le support (44, 72, 88) de l'agent aromatique est situé après l'évaporateur.

14. Dispositif selon la revendication 13, **caractérisé en ce qu'**il comprend en outre un radiateur de chauffage (32), et **en ce que** le support (44, 72, 88) de l'agent aromatique est situé après le radiateur de chauffage.

15. Dispositif selon l'une des revendications 13 et 14, **caractérisé en ce qu'**il comprend en outre des sorties d'air (12, 14, 16) débouchant dans l'habitacle du véhicule, et **en ce que** le support (44, 72, 88) de l'agent aromatique est situé avant les sorties d'air dans le véhicule.

## Claims

1. Heating and/or air conditioning device for the interior of a motor vehicle, comprising a housing (4) halving an upstream end (6) and a downstream end (10) opening into the interior, a blower (18) for drawing a flow of air into the housing (4) through the upstream end and for blowing it into the interior through the downstream end of the housing (4), odorizing means comprising a casing (40) containing a quantity of one or more aromatic agents (50) attached to a support (44, 72, 88) housed in the casing (50, 86) and capable of giving off a fragrance, the casing (50, 86) comprising an aromatic agent delivery zone pierced with at least one passageway opening (42, 92, 96) in order to allow the flow of air (56, 58) to pass through the casing,
**characterized in that** the support (44, 72, 88) of the aromatic agent can be moved between at least one delivery position in which the aromatic agent or one of the aromatic agents is facing the delivery zone, and a storage position in which the aromatic agent is isolated from the delivery zone by a barrier (52) that is sealed to the fragrance of the aromatic agent, the device comprising a control (44) which allows an operator situated in the interior of the vehicle to control the movement of the movable support (44, 72, 88) between the delivery position and the storage position.

2. Device according to Claim 1, **characterized in that** the support (44) can be moved in translation.

3. Device according to Claim 2, **characterized in that** the casing consists of a tube (40) and the movable support consists of a rod (44) mounted so as to slide in the tube (40), at least one aromatic agent (50) being mounted on the sliding rod (44) and at least one separating disc (52) forming a barrier sealed to the vapour of the aromatic agent being provided on the sliding rod (44) in order to isolate the aromatic agent or the aromatic agents from the delivery zone.

4. Device according to Claim 1, **characterized in that** the support (72) can be moved in roation.

5. Device according to Claim 4, **characterized in that** the rotating support is a disc (72) comprising a plurality of angularly spaced housings, each housing being capable of containing an aromatic agent (50).

6. Device according to Claim 5, **characterized in that** the rotating support is a cylinder (88) comprising perforations (100, 102) in two opposite zones and mounted so as to rotate about a longitudinal axis (90), this cylinder containing a quantity of at least one aromatic agent, and **in that** the casing (86) comprises at least two diametrically opposed orifices (92, 96), forming a delivery zone of the aromatic agent, the rotating support (88) being able to be moved angularly between a delivery position in which the perforations of the cylinder are facing the delivery zone of the casing, and a storage position in which the perforations of the cylinder (88) are angularly offset from the delivery zone.

7. Device according to Claim 6, **characterized in that** it comprises an air filter (80), and **in that** the casing (86) is incorporated into the air filter (80).

8. Device according to one of Claims 1 to 7, **characterized in that** the aromatic agent is in the form of a solid cartridge.

9. Device according to one of Claims 1 to 8, **characterized in that** it comprises a module (60) situated on the board panel of the vehicle and incorporating the casing (40), the latter being connected to the housing (4) via an air inlet tube (64) connected downstream of the blower (18) and via an air outlet tube (66) connected to the housing (4) downstream of the air inlet tube (64).

10. Device according to Claim 9, **characterized in that** the air outlet tube (66) is connected to a mixing zone (38) of the housing (4) of the heating device.

11. Device according to one of Claims 7 to 10, **characterized in that** the module (60) comprises a front access hole (70) that is accessible to the driver or to a passenger of the vehicle, this access hole (70) allowing the replacement of the cartridges (50) of aromatic agent.

12. Device according to one of Claims 1 to 11, **characterized in that** it comprises means (108, 110; 114) for displaying the selected aromatic agent.

13. Device according to one of Claims 1 to 12, **characterized in that** it also comprises an evaporator (30) and **in that** the support (44, 72, 88) of the aromatic agent is situated after the evaporator.

14. Device according to Claim 13, **characterized in that** it also comprises a heater (32), and **in that** the support (44, 72, 88) of the aromatic agent is situated after the heater.

15. Device according to one of Claims 13 and 14, **characterized in that** it also comprises air outlets (12, 14, 16) opening into the interior of the vehicle, and **in that** the support (44, 72, 88) of the aromatic agent is situated before the air outlets in the vehicle.

## Patentansprüche

1. Heiz- und/oder Klimasteuerungsvorrichtung für den Innenraum eines Kraftfahrzeugs mit einem Gehäuse (4), das ein stromaufwärtiges Ende (6) und ein stromabwärtiges Ende (10), das im Innenraum mündet, hat, einem Ventilator (18) zum Ansaugen eines Luftstroms in das Gehäuse (4) durch das stromaufwärtige Ende und zum Blasen des Luftstroms in den Innenraum durch das stromabwärtige Ende des Gehäuses (4) und Duftdiffusionsmitteln mit einer Aufnahme (40), die eine Menge eines oder mehrerer Aromastoffe (50) enthält, die auf einer Grundplatte (44, 72, 88) fixiert sind, die in der Aufnahme (50, 86) aufgenommen ist, und geeignet sand, einen Duft abzugeben, wobei die Aufnahme (50, 86) einen Bereich zur Diffusion des Aromastoffs umfasst, wobei der Bereich von mindestens einer Durchgangsöffnung (42, 92, 96) durchbrochen ist, um den Durchgang des Luftstroms (56, 58) durch die Aufnahme zu gestatten,
**dadurch gekennzeichnet, dass** die Grundplatte (44, 72, 88) des Aromastoffs zwischen mindestens einer Diffusionsposition, in der der Aromastoff oder einer der Aromastoffe dem Diffusionsbereich gegenüberliegt, und einer Lagerposition beweglich ist, in der der Aromastoff durch eine für den Duft des Aromastoffs dichte Sperre (52) vom Diffusionsbereich isoliert ist, wobei die Vorrichtung eine Steuerung (44) umfasst, die es einer sich im Innenraum des Fahrzeugs befindenden Bedienperson gestattet, die bewegliche Grundplatte (44, 72, 88) zwischen der Diffusionsposition und der Lagerposition zu verschieben.

2. Vorrichtung nach Anspruch 1, **dadurch gekennzeichnet, dass** die Grundplatte (44) translatorisch beweglich ist.

3. Vorrichtung nach Anspruch 2, **dadurch gekennzeichnet, dass** die Aufnahme aus einer Röhre (40) und die bewegliche Grundplatte aus einer Stange (44) besteht, die gleitend in der Röhre (40) angebracht ist, wobei mindestens ein Aromastoff (50) an der gleitenden Stange (44) angebracht ist und mindestens eine Trennscheibe (52), die eine gegenüber dem Dampf des Aromastoffs dichte Sperre bildet, an der gleitenden Stange (44) vorgesehen ist, um den Aromastoff oder die Aromastoffe vom Diffusionsbereich zu isolieren.

4. Vorrichtung nach Anspruch 1, **dadurch gekennzeichnet, dass** die Grundplatte (72) drehbeweglich ist.

5. Vorrichtung nach Anspruch 4, **dadurch gekennzeichnet, dass** es sich bei der sich drehenden Grundplatte um eine Scheibe (72) handelt, die mehrere winkelmäßig beabstandete Aufnahmen umfasst, die jeweils geeignet sind, einen Aromastoff (50) zu enthalten.

6. Vorrichtung nach Anspruch 5, **dadurch gekennzeichnet, dass** es sich bei der sich drehenden Grundplatte um einen Zylinder (88) handelt, der Perforationen (100, 102) in zwei gegenüberliegenden Bereichen umfasst und sich um eine Längsachse (90) drehend angebracht ist, wobei dieser Zylinder eine Menge mindestens eines Aromastoffs enthält, und dass die Aufnahme (86) mindestens zwei diametral gegenüberliegende Öffnungen (92, 96) umfasst, die einen Bereich zur Diffusion des Aromastoffs darstellen, wobei die sich drehende Grundplatte (88) winkelmäßig zwischen einer Diffusionsposition, in der die Perforationen des Zylinders dem Diffusionsbereich der Aufnahme gegenüberliegen, und einer Lagerposition, in der die Perforationen des Zylinders (88) winkelmäßig zum Diffusionsbereich versetzt sind, verschoben werden kann.

7. Vorrichtung nach Anspruch 6, **dadurch gekennzeichnet, dass** sie einen Luftfilter (80) umfasst und dass die Aufnahme (86) in den Luftfilter (80) integriert ist.

8. Vorrichtung nach einem der Ansprüche 1 bis 7, **dadurch gekennzeichnet, dass** der Aromastoff in der Form einer festen Patrone vorliegt.

9. Vorrichtung nach einem der Ansprüche 1 bis 8, **dadurch gekennzeichnet, dass** sie ein Modul (60) umfasst, das im Armaturenbrett des Fahrzeugs angeordnet ist und in das die Aufnahme (40) eingegliedert ist, wobei Letztere über ein Lufteingangsrohr (64), das stromabwärts an den Ventilator (18) angeschlossen ist, und über ein Luftausgangsrohr (66), das stromabwärts des Lufteingangsrohrs (64) an das Gehäuse (4) angeschlossen ist, mit dem Gehäuse (4) verbunden ist.

10. Vorrichtung nach Anspruch 9, **dadurch gekennzeichnet, dass** das Luftausgangsrohr (66) mit einem Mischbereich (38) des Gehäuses (4) der Heizvorrichtung verbunden ist.

11. Vorrichtung nach einem der Ansprüche 7 bis 10, **dadurch gekennzeichnet, dass** das Modul (60) ein Zugriffsloch (70) in der Blende umfasst, auf das der Fahrer oder ein Fahrzeuginsasse zugreifen kann, wobei das Zugriffsloch (70) den Austausch der Aromastoffpatronen (50) gestattet.

12. Vorrichtung nach einem der Ansprüche 1 bis 11, **dadurch gekennzeichnet, dass** sie Mittel (108, 110; 114) zur Anzeige des gewählten Aromastoffs umfasst.

13. Vorrichtung nach einem der Ansprüche 1 bis 12, **dadurch gekennzeichnet, dass** sie ferner einen Verdampfer (30) umfasst und dass die Grundplatte (44, 72, 88) des Aromastoffs nach dem Verdampfer angeordnet ist.

14. Vorrichtung nach Anspruch 13, **dadurch gekennzeichnet, dass** sie ferner einen Wärmetauscher (32) umfasst und dass die Grundplatte (44, 72, 88) des Aromastoffs nach dem Wärmetauscher angeordnet ist.

15. Vorrichtung nach Anspruch 13 oder 14, **dadurch gekennzeichnet, dass** sie ferner Luftaustritte (12, 14, 16) umfasst, die im Innenraum des Fahrzeugs münden, und dass die Grundplatte (44, 72, 88) des Aromastoffs vor den Luftaustritten im Fahrzeug angeordnet ist.
